# EUROPEAN PATENT APPLICATION

(11) **EP 2 575 263 A1**
(43) Date of publication of application: **03.04.2013**
(21) Application number: 12184734.7
(22) Date of filing: 17.09.2012
(51) Int. Cl.: H04B 3/56, A61B 5/00

(54) **Hospital bed having powerline communication capability**

(30) Priority: 20.09.2011 US 201113236741
(71) Applicant: Hill-Rom Services, Inc., Batesville, IN 47006-9167 (US)
(72) Inventor: Frondorf, Michael M., Lakeside Park, KY Kentucky 41017 (US)
(74) Representative: Findlay, Alice Rosemary

(57) **Abstract**

Apparatus for communicating with a nurse call system is provided. The apparatus includes a hospital bed and a module spaced from the hospital bed. The hospital bed has a power cord including a first AC plug that plugs into an AC receptacle of the module. The module has a separate power cord that plugs into a standard AC power receptacle. Thus, power is provided to the hospital bed via the module. Bed data is communicated to the module via at least one conductor of the power cord that couples the bed to the module. The module has circuitry that extracts the bed data sent from the bed via the power cord and communicates the bed data to the nurse call system.

## Description

The present disclosure relates to hospital beds, and particularly, to hospital beds that are communicatively linked to nurse call systems. More particularly, the present disclosure relates to the components associated with transfer of data between a hospital bed and a nurse call system.

Hospital beds that communicate with nurse call systems are known. Such hospital beds typically have a communication cable, such as the 37-pin style of cable found in many hospital beds marketed by Hill-Rom Company, Inc. The communication cable is separate from the power cord of most prior art hospital beds. Thus, to move a hospital bed from one location to another, both the communication cable and the power cord need to be disconnected from a respective communication port and power receptacle. Caregivers sometimes forget to disconnect the communication cable prior to moving the hospital bed which results, in many instances, to damaging the communication cable.

The present invention comprises an apparatus, system and/or method, or a component or step thereof, that has any one or more of the following features alone or in any combination.

An apparatus for communicating with a nurse call system may include a hospital bed which may have a power cord including a first AC plug. The hospital bed may have bed circuitry that may send bed data over at least one conductor of the power cord. The apparatus may further include a module that may be spaced from the hospital bed. The module may have a second AC plug that couples to a standard AC power receptacle in a room of a healthcare facility. The module may further have a module AC power receptacle to which the first AC plug of the power cord of the hospital bed couples to receive power. The module may have module circuitry that may be coupled to the at least one conductor of the power cord and that extracts the bed data. The module may also have an output device that may be configured to couple the module circuitry to the nurse call system for transfer of the bed data from the module circuitry to the nurse call system.

In some embodiments, the module circuitry may include a low pass filter interposed between the second AC plug and the module AC power receptacle to block powerline communications from being communicated to the second plug. Alternatively or additionally, the module circuitry may include isolation circuitry to isolate the bed data from being communicated to a power grid of a healthcare facility that delivers power to the standard AC power receptacle while permitting the bed data to be communicated to the nurse call system.

According to this disclosure, the output device may include, for example, an outlet port and/or a communication cable, such as a 37 pin connector. In some embodiments, the module includes at least one speaker. The at least one speaker also may serve as a microphone. The at least one speaker may be used for voice communication from the nurse call system, for example. The at least one speaker may also be used for television sound. It is contemplated by this disclosure that the at least one speaker may comprise a pair of speakers. In such embodiments, each speaker of the pair of speakers may also serve as a microphone.

The bed data sent over the at least one conductor of the power cord may comprise serial data. Data from the nurse call system may be communicated to the bed circuitry via the module and the at least one conductor of the power cord such that the bed may communicate bidirectionally over the power cord. The bed data may include bed identification (ID) data and the module may have module identification (ID) data associated therewith. The bed ID data and the module ID data may be transmitted by the module to the nurse call system. The nurse call system may then use the bed ID data and module ID data to associate the hospital bed with the module.

Alternatively or additionally, a pillow speaker unit may be communication with the module circuitry. The bed data may include data pertaining to one or more of the following: a position of one portion of a bed frame relative to another portion of the bed frame, a mattress function, a status of a bed exit alarm system of the hospital bed, and patient physiologic data.

The invention will now be further described by way of example with reference to the accompanying drawings, in which:

Fig. 1 is a block diagram showing a system including a hospital bed, a wall module spaced from the hospital bed, and a nurse call system, the wall module plugs into a standard AC power receptacle of a power grid of a healthcare facility, the bed has a power cord that plugs into an AC receptacle of the module to receive power, and bed data is communicated over the power cord to the module which, in turn, communicates the bed data to the nurse call system;

Fig. 2 is a block diagram showing blocks of electrical circuitry of the hospital bed; and

Fig. 3. is a block diagram showing blocks of electrical circuitry of the wall module.

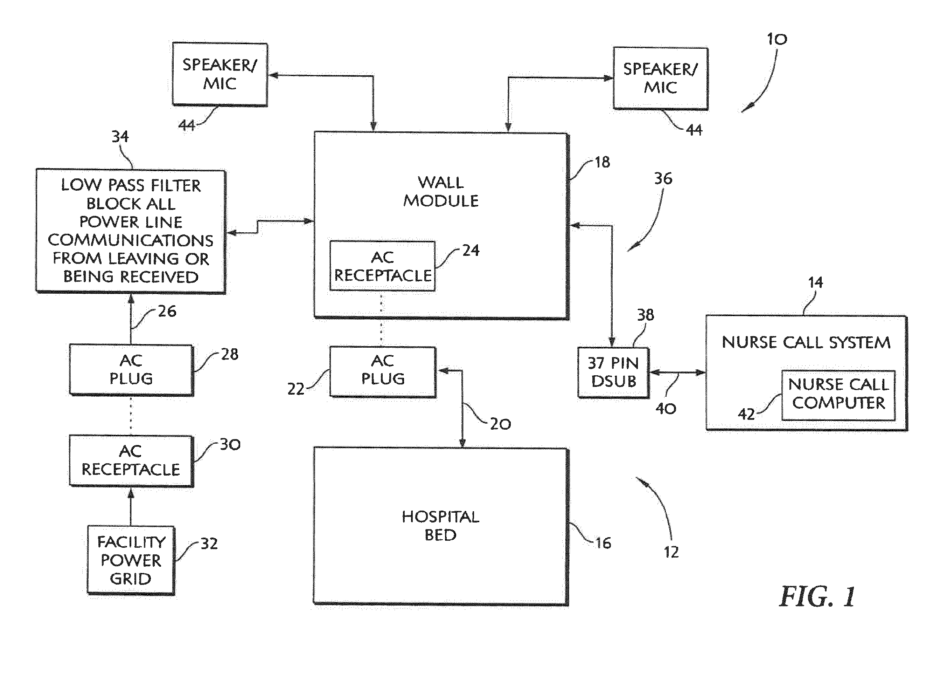

A system 10 according to this disclosure includes apparatus 12 configured for communication with a nurse call system 14 as shown diagrammatically in Fig. 1. Apparatus 12 includes a hospital bed 16 and a wall module 18. Bed 16 has a power cord 20 which terminates at an AC plug 22. Module 18 has an AC receptacle 24 to which plug 22 couples. Module 18 has its own power cord 26 that terminates at an AC plug 28 which is coupleable to an AC receptacle 30 of a power grid 32 of a healthcare facility.

According to this disclosure, bed 16 sends and receives data over at least one of the conductors of power cord 20. Thus, bed 16 has powerline communication capability. A low pass filter 34 blocks any data sent to bed 16 or sent from bed 16 from reaching power cord 26, plug 28, and receptacle 30 thereby keeping the facility power grid 32 isolated from the bidirectional communications to/from bed 16. Module 18 includes an output device 36 such as a 37 pin Dsub 38 which couples to nurse call system 14 such as via a nurse call cable 40 as indicated diagrammatically in Fig. 1. Nurse call system 14 includes a nurse call computer 42 as well as other communication equipment such as one or more bed interface units (BIU's), network interface units (NIU's), and audio stations (not shown) that are located in the room with bed 16. Examples of the type of communication equipment included in various embodiments of nurse call system 14 (as well as system 10, in general) can be found in U.S. Patent Nos. 7,746,218; 7,538,659; 7,319,386; 7,242,308; 6,897,780; 6,362,725; 6,147,592; 5,838,223; 5,699,038 and 5,561,412 and U.S. Patent Application Publication Nos. 2009/0217080; 2009/0214009; 2009/0212956; and 2009/0212925.

Nurse call system 14 includes devices that are capable of voice communications with a patient situated on hospital bed 16. For example, computer 42 is situated at a master nurse station and includes a telephone in some embodiments. One or more speakers 44 are coupled to or included in module 18 as indicated diagrammatically in Fig. 1. It is contemplated by this disclosure that speakers 44 also may serve as microphones. Accordingly, the text "speaker/mic" appears in each of blocks 44. In the illustrative example, a pair of speaker/mics 44 are included with module 18. Alternatively or additionally, one of speaker/mics 44 are provided as part of a pillow speaker. A pillow speaker is a handheld unit that a patient uses while in bed to place a nurse call or to control a television, for example. Pillow speaker units typically have a fairly long cable that plugs into a wall outlet. Thus, according to this disclosure, module 18 has an outlet for a plug or coupler at the end of pillow speaker cable in some embodiments.

Regardless of whether speaker/mics 44 are situated on or within a housing of module 18, are mounted separately on a room wall or some other structure coupled to a room wall, or are included as part of a pillow speaker, the speaker/mics 44 are used by the patient during voice communication with a caregiver at the master nurse station associated with computer 42. Thus, according to this disclosure, voice communications are not included among the data that is transmitted to/from bed 12 via the one or more conductors of power cord 20 in some embodiments. In other embodiments, one or more speaker/mics, similar to speaker/mics 44, are provided on bed 16 and bidirectional voice communication data is among the data transmitted to/from bed 16 on one or more conductors of power cord 20.

Examples of the type of bed data that is transmitted from bed 16, for various embodiments of bed 16, is summarized below in Table 1 as follows:

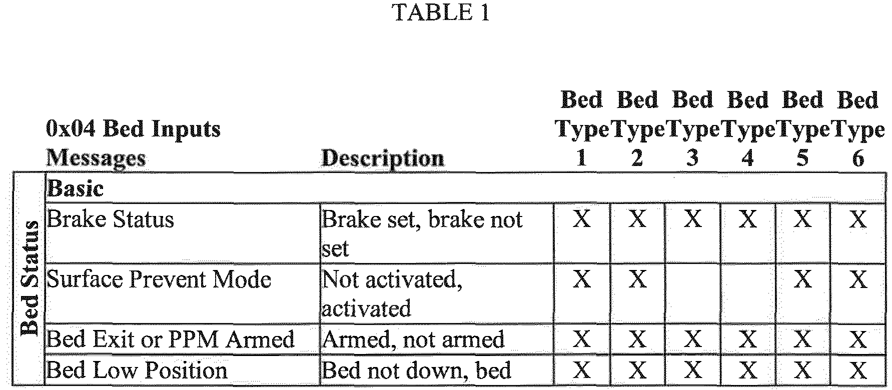

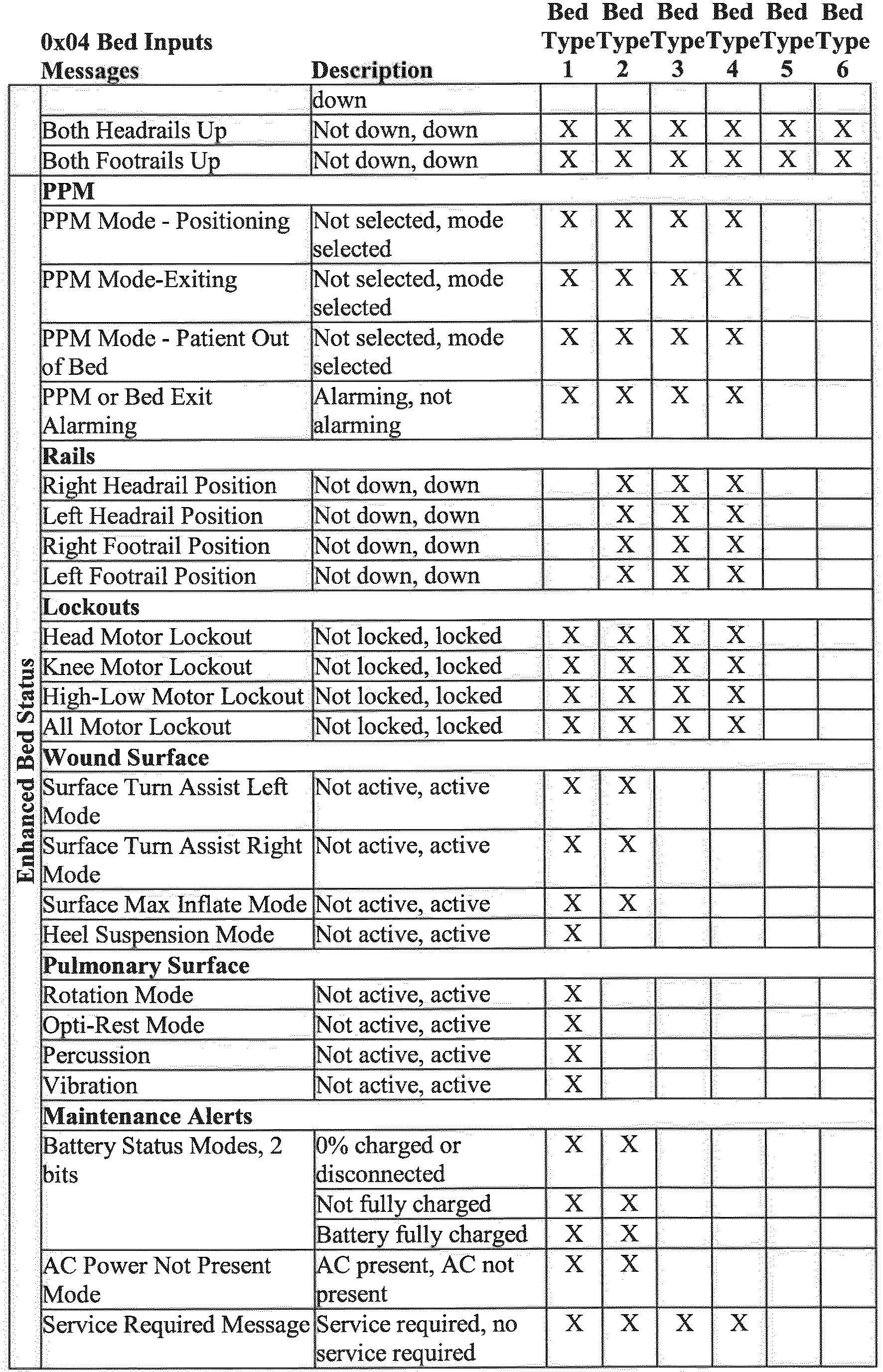

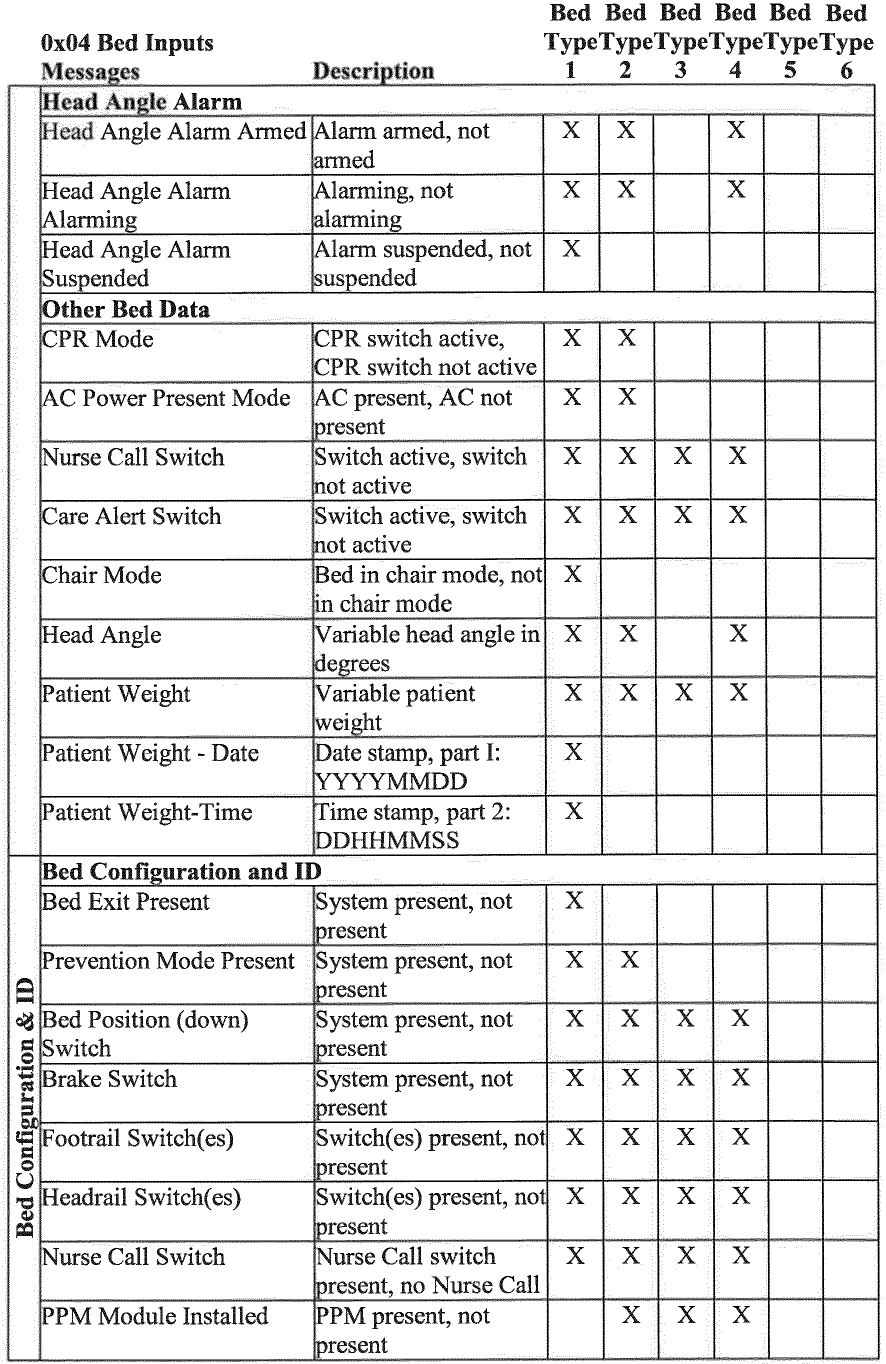

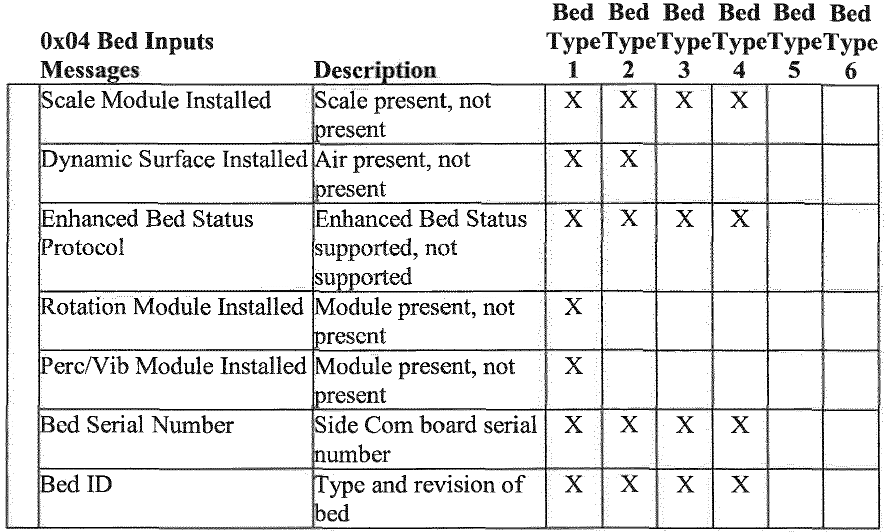

In the example of Table 1, Bed Type 1 is the TOTALCARE® bed, Bed Type 2 is the VERSACARE® bed, Bed Type 3 is the CAREASSIST® ES bed, Bed Type 4 is the ADVANTA™ 2 bed, Bed Type 5 is the ADVANCE bed, and Bed Type 6 is the ADVANTA bed, each of which is, or was, marketed by Hill-Rom Company, Inc. Beds 16 of other types which have other types of bed data are, of course, within scope of this disclosure. Based on Table 1, it will be appreciated that bed data includes, for example, data pertaining to one or more of the following: a position of one portion of a bed frame relative to another portion of the bed frame (e.g., Brake Status, Bed Low Position, Rail positions, Head Angle), a mattress function (e.g., Wound Surface and Pulmonary Surface information), a status of a bed exit alarm system of the hospital bed (e.g., the patient position monitoring (PPM) information), and patient physiologic data (e.g., patient weight).

In some contemplated embodiments, bed 16 has a mattress and/or bed frame with sensors to sense patient physiologic data (e.g., heart rate, temperature, respiration rate, blood oxygenation, blood pressure, etc.) and that such data is also among the bed data communicated from bed 16 to module 18 and then on to computer 42 via power cord 20. An example of a mattress with physiologic sensors can be found in U.S. Patent Nos. 7,515,059; 7,330,127 and 6,721,980. Other examples of mattresses and bed frames having physiologic sensors can be found in U.S. Patent Application Publication No. 2010/0101022.

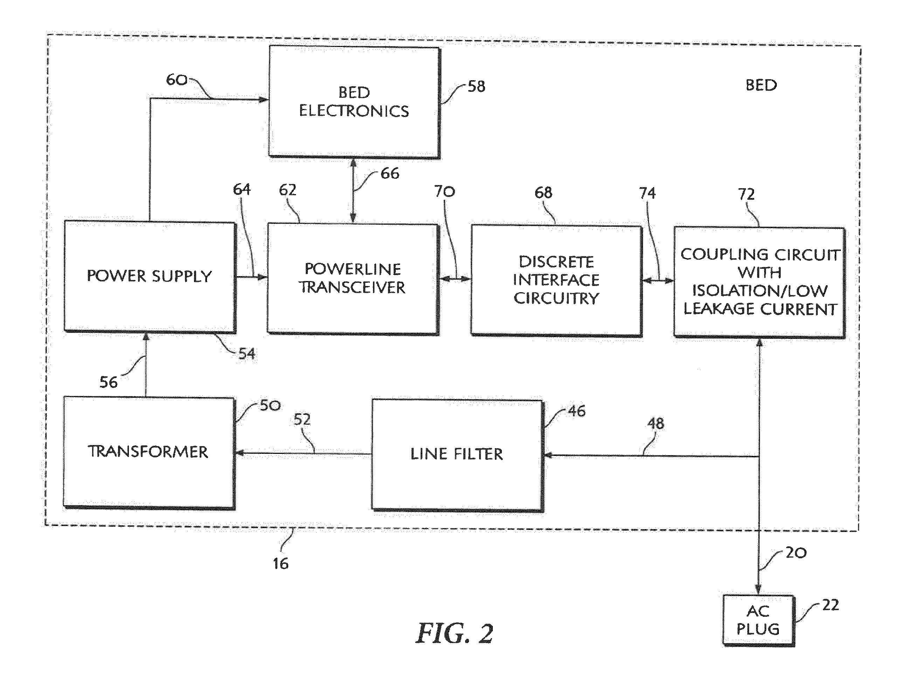

Referring now to Fig. 2, bed 16 has a line filter 46 with an input coupled to conductors of power cord 20 as indicated by line 48. An output of line filter 46 is coupled to an input of a transformer 50 as indicated by line 52. An output of transformer 50 is coupled to an input of power supply 54 as indicated by line 56. Transformer 52 reduces down the amplitude of the incoming 120 Volt (V) alternating current (AC) power signal and provides electrical isolation from the high voltage mains power. Power supply 54 converts the stepped down AC power signal to the direct current (DC) voltage levels required for the remaining electrical components of bed 16. For example, in some embodiments, power supply 54 includes one or more rectifiers and one or more voltage converters to produce 5 V and 12 V that are typically used in the operation of solid state devices. Power supply 54 also includes, for example, the circuitry that provides power to operate bed elevation motors and bed deck articulation motors which oftentimes run on 24 V AC signals. In some embodiments, power supply 54 also includes any batteries and battery recharging circuitry of bed 16. So, for purposes of this disclosure, suffice it to say that power supply 54 includes all of the circuitry that receives incoming mains power to bed 16 and that converts it to the various voltage levels needed to run other components of bed 16.

Power supply 54 includes one output coupled to bed electronics 58 as indicated by line 60 and another output coupled to a powerline transceiver 62 as indicated by line 64. Bed electronics 58 is also coupled to powerline transceiver 62 for bidirectional communication as indicated by line 66. Block 58 in Fig. 2 is intended to represent all of the bed electronics in a hospital bed other than the components otherwise depicted diagrammatically in Fig. 2. Thus, bed electronics 58 includes user inputs such as buttons, switches, and graphical display screens, as well as other electrical components such as sensors, processors, memory devices, and the discrete circuit components associated with these. Examples of powerline transceiver 62 include frequency shift keying modulators/demodulators (MODEM's) such as those available from STMicroelectronics (e.g., Model Nos. ST7538Q and ST7540), those available from Maxim Integrated Products (e.g., Chipset Model Nos. MAX2981/MAX2986, MAX2990/MAX2991, and MAX2992/MAX2991), and those available from Cypress Semiconductor Corporation (e.g., Model Nos. CY8CPLC20 and CYBCLED16P01) just to name a few.

In the illustrative example of Fig. 2, powerline transceiver 62 is coupled to discrete interface circuitry 68 for bidirectional communication as indicated by line 70. Discrete interface circuitry 68 is coupled to a coupling circuit 72 with isolation and/or low leakage current for bidirectional communication as indicated by line 74. Circuit 72 is coupled to the one or more conductors of cord 20 for bidirectional communication.

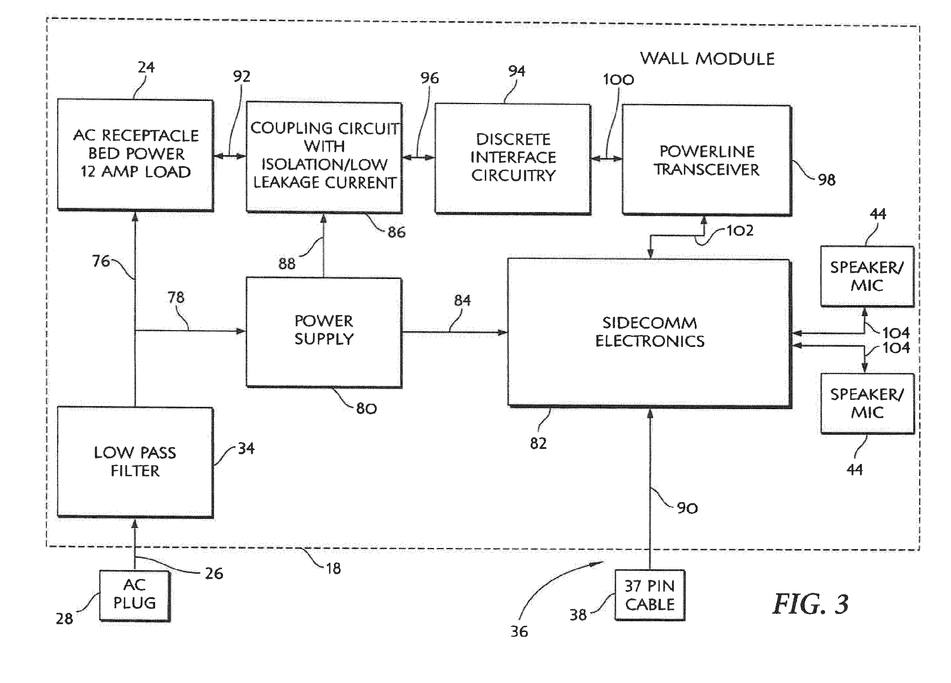

Referring now to Fig. 3, module 18 has a low pass filter 34 with an input coupled to conductors of a power cord associated with plug 28 as indicated by line 26. An output of low pass filter 34 is coupled to AC receptacle 24 from which bed 16 receives power sufficient to support a 12 amp load as indicated by line 76. The output of low pass filter 34 is also coupled to an input of power supply 80 as indicated by line 78. Power supply 80 converts the AC power signal to the direct current (DC) voltage levels required for the remaining electrical components of module 18. For example, in some embodiments, power supply 80 includes one or more rectifiers and one or more voltage converters to produce 5 V and 12 V that are typically used in the operation of solid state devices.

Power supply 80 includes one output coupled to sidecomm electronics 82 as indicated by line 84 and another output coupled to a coupling circuit 86 as indicated by line 88. Sidecomm electronics 82 of module 18 are fashioned substantially similarly, or identically, to the sidecomm electronics of a hospital bed. The sidecomm electronics of a hospital bed are those electronics associated with communication of signals to or from the devices oftentimes included on one or more siderails of the bed. Thus, the sidecomm electronics 82 of module 18 serve as an emulator of at least a portion of the electronics of a hospital bed, such as the bed electronics 58 discussed herein. Thus, from the perspective of nurse call system 14, the output device 36 of module 18 which is coupled to sidecomm electronics 82 as indicated by line 90, communicates data to the nurse call system 14 and receives data from the nurse call system 14 in the same manner as if nurse call system 14 were connected directly to the hospital bed via a data cable, for example.

Coupling circuit 86 includes circuitry for isolation and/or low leakage current and communicates bidirectionally with AC receptacle 24 as indicated by line 92. Coupling circuitry 86 is coupled to discrete interface circuitry 94 for bidirectional communication as indicated by line 96. Discrete interface circuitry 68 is also coupled to a powerline transceiver 98 for bidirectional communication as indicated by line 100. Powerline transceiver 98 is, in turn, coupled for bidirectional communication with sidecomm electronics 82 as indicated by line 102. Powerline transceiver 98 of module 18 has substantially similar or identical features to those of powerline transceiver 62 of bed 16 described above. In the illustrative example of Fig. 3, speaker/mics 44 are each located within the housing of module 18 (as suggested by the location of blocks 44 within the dotted line perimeter of module 18 in Fig. 3) and are coupled to sidecomm electronics 82 for bidirectional communication as indicated by respective lines 104.

According to this disclosure, computer 42 associates bed 16 and module 18 with each other based on bed identification (ID) data transmitted by bed 16 and based on module ID data transmitted by module 18. Bed ID data is transmitted by bed 16 over power cord 20 to module 18 and then module 18 transmits both the bed ID data and the module ID data to computer 42 of system 14 via output device 36. By providing bed data over the same power cord 20 from which bed 16 receives its power, the need for a second data cable is eliminated. However, the wired connection of power cord 20 to module 18 via plugging plug 22 into receptacle 24, permits computer 42 to make a positive association between bed 16 and module 18 and ultimately, therefore, to a room location at which module 18 is located. This wired connection between bed 16 and module 18 via power cord 20, therefore, eliminates the sorts of problems that can be introduced with associating devices that communicate wirelessly. For example, wireless communication by infrared (IR) signals requires a clean line of sight between IR transmitters and receivers which can be blocked or otherwise interrupted by other items and equipment. As another example, wireless communication by radio frequency (RF) signals can pass through walls, floors, and ceilings such that a receiver may receive RF transmissions from a multitude of devices instead of a single device that is to be associated with the receiver.

Although nurse call system 14 and nurse call computer 42 are shown in Fig. 1, it is within the scope of this disclosure for apparatus 12 to be used in connection with one or more other types of systems in a healthcare facility. Thus, alternatively or additionally, apparatus 12 may be communicatively coupled to an electronic medical records (EMR) system, an admission/discharge/transfer (ADT) system, a pharmacy system, a laboratory system, a billing and payment system, an insurance system, and so forth. That is, apparatus 12 of the present disclosure is capable of communicating with any type of remote computer 42 via network infrastructure of a factility using the principles disclosed herein.

Although certain illustrative embodiments have been described in detail above, variations and modifications.

## Claims

1. Apparatus for communicating with a nurse call system, the apparatus comprising
a hospital bed having a power cord including a first AC plug, the hospital bed having bed circuitry that sends bed data over at least one conductor of the power cord, and
a module spaced from the hospital bed, the module having a second AC plug that couples to a standard AC power receptacle in a room of a healthcare facility, a module AC power receptacle to which the first AC plug of the power cord of the hospital bed couples to receive power, module circuitry that is coupled to the at least one conductor of the power cord and that extracts the bed data, and an output device configured to couple the module circuitry to the nurse call system for transfer of the bed data from the module circuitry to the nurse call system.

2. The apparatus of claim 1, wherein the module circuitry comprises a low pass filter interposed between the second AC plug and the module AC power receptacle to block powerline communications from being communicated to the second plug.

3. The apparatus of either claim 1 or claim 2, wherein the module circuitry includes isolation circuitry to isolate the bed data from being communicated to a power grid of a healthcare facility that delivers power to the standard AC power receptacle while permitting the bed data to be communicated to the nurse call system.

4. The apparatus of any preceding claim, wherein the output device comprises an outlet port.

5. The apparatus of any one of claims 1 to 3, wherein the output device of the module comprises a communication cable.

6. The apparatus of claim 5, wherein the communication cable comprises a 37 pin connector.

7. The apparatus of any preceding claim, wherein the module includes at least one speaker.

8. The apparatus of claim 7, wherein the at least one speaker also serves as a microphone.

9. The apparatus of either claim 7 or claim 8, wherein the at least one speaker is used for voice communication from the nurse call system, and/or the at least one speaker is also used for television sound.

10. The apparatus of any one of claims 7 to 9, wherein the at least one speaker comprises a pair of speakers.

11. The apparatus of any preceding claim, wherein the bed data sent over the at least one conductor of the power cord comprises serial data.

12. The apparatus of any preceding claim, wherein data from the nurse call system is communicated to the bed circuitry via the module and the at least one conductor of the power cord such that the bed communicates bidirectionally over the power cord.

13. The apparatus of any preceding claim, wherein the bed data includes bed identification (ID) data and the module has module identification (ID) data associated therewith, the bed ID data and the module ID data is transmitted by the module to the nurse call system, and the nurse call system uses the bed ID data and module ID data to associate the hospital bed with the module.

14. The apparatus of any preceding claim, further comprising a pillow speaker unit in communication with the module circuitry.

15. The apparatus of any preceding claim, wherein the bed data comprises data pertaining to a position of one portion of a bed frame relative to another portion of the bed frame, and/or data pertaining to a mattress function, and/or data pertaining to a status of a bed exit alarm system of the hospital bed, and/or patient physiologic data.
